# EUROPEAN PATENT SPECIFICATION

(11) **EP 1 379 242 B1**
(45) Date of publication and mention of the grant of the patent: **15.08.2012**
(21) Application number: 02713920.3
(22) Date of filing: 20.03.2002
(51) Int. Cl.: A61K 31/4164, A61K 31/4184, A61P 35/00, A61K 47/44

(54) **METHOD FOR TREATMENT OF CANCER AND COMPOSITIONS FOR USE THEREIN**
VERFAHREN ZUR BEHANDLUNG VON KREBS UND DAFÜR VERWENDBARE ZUSAMMENSETZUNGEN
METHODE DE TRAITEMENT DE CANCERS ET COMPOSITIONS APPROPRIEES

(30) Priority: 26.03.2001 US 278435 P; 30.03.2001 CA 2342470
(43) Date of publication of application: 14.01.2004
(73) Proprietor: NEWSOUTH INNOVATIONS PTY LIMITED, Kensington NSW 2052 (AU)
(72) Inventor: MORRIS, David, Lawson, Lugarno, New South Wales 2210 (AU); POURGHOLAMI, Mohammad, Hossein, Penshurst, New South Wales, 222 (AU)
(74) Representative: Brasnett, Adrian Hugh
(86) International application number: PCT/AU2002/000339
(87) International publication number: WO 2002/076454

(56) References cited:
- WO-A-00/41669
- WO-A-01/12169
- WO-A-01/17504
- WO-A1-00/15766
- HOSSEIN POURGHOLAMI M ET AL: "IN VITRO INHIBITION OF HUMAN LIVER CANCER CELLS BY ALBENDAZOLE" PROCEEDINGS OF THE ANNUAL MEETING OF THE AMERICAN ASSOCIATION FOR CANCER RESEARCH, NEW YORK, NY, US, vol. 41, 5 April 2000 (2000-04-05), page 656, XP001179562 ISSN: 0197-016X
- TADA Y ET AL: "Acute renal toxicity of thiabendazole (TBZ) in ICR mice" FOOD AND CHEMICAL TOXICOLOGY, vol. 30, no. 12, 1992, pages 1021-1030, XP002447229 ISSN: 0278-6915
- LUNDY J. ET AL: 'Chemoimmunotherapy of a murine fibrosarcoma: Critical factors for success of combined modality therapy' J. OF SURGICAL ONCOLOGY vol. 9, no. 4, 1977, pages 339 - 345, XP002972580
- ROLIN S. ET AL: 'Study of the in vitro bioactivation of albendazole in human liver microsomes and hepatoma cell lines' CELL BIOLOGY & TOXICOLOGY vol. 5, no. 1, 1989, pages 1 - 14, XP002972581
- LANNUSSE C.E.: 'Influence on the antithyroid compound methimazole on the plasma disposition of fenbendazole and oxfendazole in sheep' RESEARCH IN VET. SCIENCE vol. 58, 1995, pages 222 - 226, XP002972582
- DE BRABANDER M.J. ET AL: 'The effects of methyl (5-(2-thienylcarbonyl)-1H-benzimidazol-2-yl )carbamate, (R 17934; NSC238159), a new synthetic antitumoral drug interfering with microtubules, on mammalian cell cultured in vitro' CANCER RESEARCH vol. 36, 1976, pages 905 - 916, XP002972583
- MORRIS D.L. ET AL: 'Pilot study of albendazole in patients with advanced malignancy' ONCOLOGY vol. 61, July 2001, pages 42 - 46, XP002972584
- POURGHOLAMI M.H. ET AL: 'In vitro and in vivo suppression of growth of hepatocellular carcinoma cells by albendazole' CANCER LETTERS vol. 165, 10 April 2001, pages 43 - 49, XP002972585
- WANG X ET AL: "Significance of MAD2 expression tot mitotic checkpoint control in ovarian cancer cells", CANCER RESEARCH, AMERICAN ASSOCIATION FOR CANCER REREARCH, US, vol. 62, 15 March 2002 (2002-03-15), pages 1662-1668, XP002989655, ISSN: 0008-5472, DOI: DOI:10.1007/0-306-47822-6_1
- MAKHIJA S ET AL: "Intraperitoneal chemotherapy strategies in the treatment of epithelial ovarian carcinoma.", CURRENT OPINION IN OBSTETRICS & GYNECOLOGY FEB 1999 LNKD- PUBMED:10047959, vol. 11, no. 1, February 1999 (1999-02), pages 23-27, ISSN: 1040-872X
- MARKMAN M: "Is there a role for intraperitoneal chemotherapy in the management of ovarian cancer?", ONCOLOGY (WILLISTON PARK, N.Y.) JAN 2001 LNKD- PUBMED:11271985, vol. 15, no. 1, January 2001 (2001-01), pages 93-98 ; disc, ISSN: 0890-9091
- "The Merck Manual, seventeeth edition", 1 June 1999 (1999-06-01)

## Description

### FIELD OF THE INVENTION

The present invention is concerned with compositions for the treatment of tumors, particularly the treatment of ovarian cancer.

### BACKGROUND OF THE INVENTION

Hepatocellular carcinoma (HCC; hepatoma) is one of the most common malignancies and a leading cause of death worldwide (1-3). Untreated, HCC typically has a dismal prognosis. Surgical resection remains the mainstay for treatment of HCC and provides the only consistent long-term tumor-free survival (4). However, resection has been limited primarily by low resectibility rates and recurrent disease. Systemic chemotherapy as a primary treatment modality for HCC has limited value because only a small portion of patients will obtain meaningful palliation with the presently available drugs and regimens (2, 4, 5,) and because the toxicity of currently available chemotherapeutic agents often outweighs their limited benefits (6). Furthermore, liver is the most common site for metastases of colorectal carcinoma which in itself is the leading cause of cancerous death in nonsmokers in the developed world (7).

Albendazole (ABZ; methyl 5-propylthio-1H-benzimidazole-2-yl carbamate) is a benzimidazole carbamate (BZs) anthelmintic developed as a veterinary product in 1975. The BZs are now important broad-spectrum drugs for the control of helminth parasites in mammals. They are effective against lungworms and gastrointestinal nematodes, tapeworms and liver flukes (8). The intrinsic anthelmintic action of benzimidazole compounds on parasite relies on a progressive disruption of basic cell functions as a result of their binding to parasite tubulin and depolymerization of microtubules. However, a number of other mechanisms including disruption of glucose uptake and metabolism have also been described for these compounds (9-11).

Pourgholumi et al. (Cancer Letters 2001, 165, 43-49) describes the in vitro and in vivo suppression of grouth of hepatocellular carcinomer cells by albendazole. The use of albendazole to treat ovarian cancer is not described.

### SUMMARY OF THE INVENTION

The present inventors tested BZs and particularly albendazole against a range of liver (HepG2, Hep3B, PLC/PRF/5, SKHEP-1, Hep1-6, HTC, Novikoff ) and colorectal cancer (C-170, HT-29 and LOVO) cell lines. The results obtained show potent and dose dependent inhibition of proliferation of these cells by albendazole (and several other BZs). Albendazole was effective against all human and animal cell lines examined, and over a 5 day treatment period, [3H]thymidine incorporation was reduced by over 80% (range 81.6 - 99.4%) in all these cell lines.

Accordingly, the present invention provides a pharmaceutical composition for use in a method of treatment, as described in claim 1.

### DESCRIPTION OF FIGURES

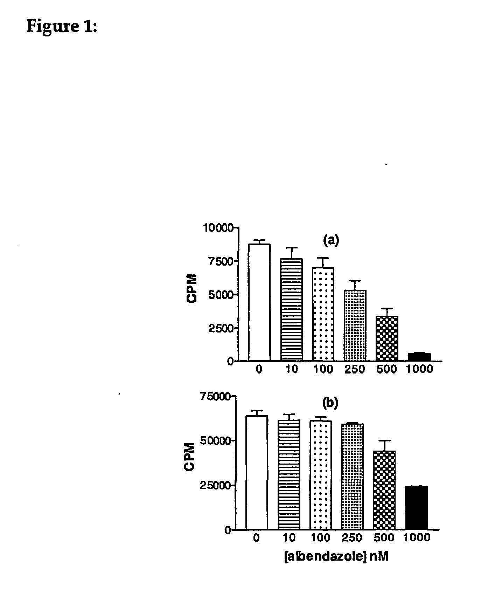

**Fig. 1****.** These figures are presented together with reference examples, for the useful understanding of the present invention. [³H] Thymidine incorporation [expressed as counts per minute (CPM)] in SKHEP-1 cells was measured either (a) immediately after 1 day treatment with albendazole or (b) after 1 day treatment with albendazole followed by 4 days treatment with the medium alone (not containing the drug). Data points are the mean ± s.e.m.

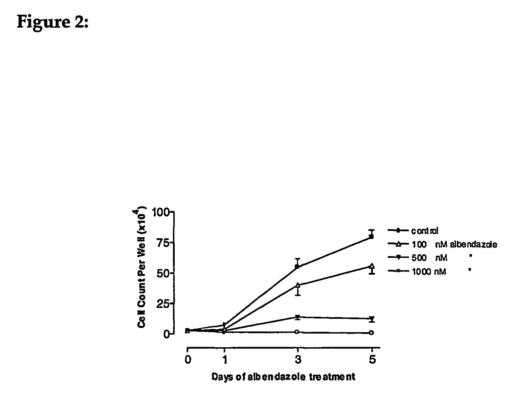

**Fig. 2****.** These figures are presented together with reference examples, for the useful understanding of the present invention. Time-course effects of albendazole on SKHEP-1 cell number. Cells growing in 6 well plates were treated for 1, 3, or 5 days with albendazole (0, 100, 500 or 1000 nM) and number of viable cells were counted using the Trypan blue exclusion method. Data points are the mean ± s.e.m.

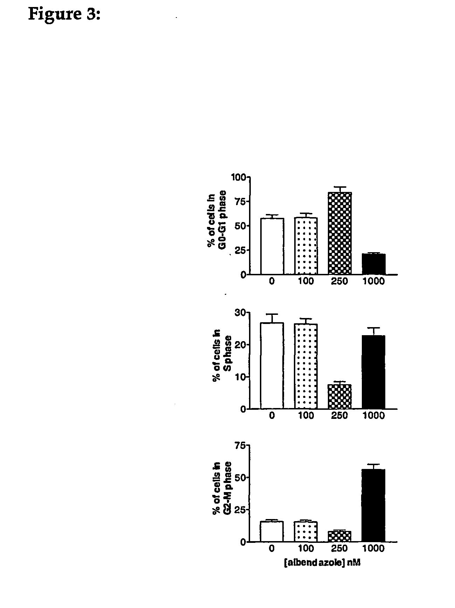

**Fig. 3****.** These figures are presented together with reference examples, for the useful understanding of the present invention. Effect of albendazole on cell cycle stage of SKHEP-1 cells. Cells were treated with different concentrations of albendazole (0, 100, 250, and 1000 nM) for 3 days, stained with propidium iodide and analyzed for DNA content by flow cytometry. A total of 10/000 nuclei were analyzed from each sample. Data points are the mean ± s.e.m. of the percentage of cells within G0-G1, S and G2-M phases of the cell cycle.

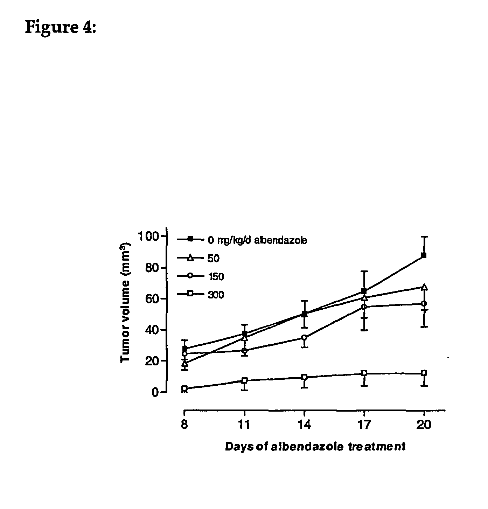

**Fig. 4****.** These figures are presented together with reference examples, for the useful understanding of the present invention. Effect of different doses of albendazole (0, 50, 150 & 300 mg/kg/ day in two divided dose given orally in sesame oil) on SKHEP-1 subcutaneous tumor formation and growth in nude mice. Changes in tumor volumes were measured every 3 days. Each value represents mean *± s.e.m. of 10 animals.

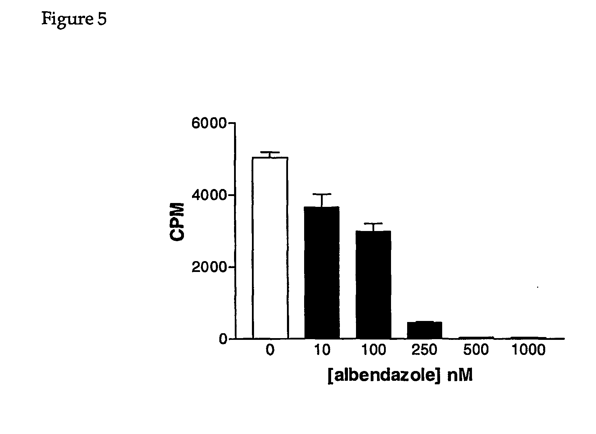

**Fig. 5** Concentration dependent inhibition of 3H-thymidine uptake (proliferation) of the ovarian cancer cell line (OVCAR-3) by albendazole *in vitro.*

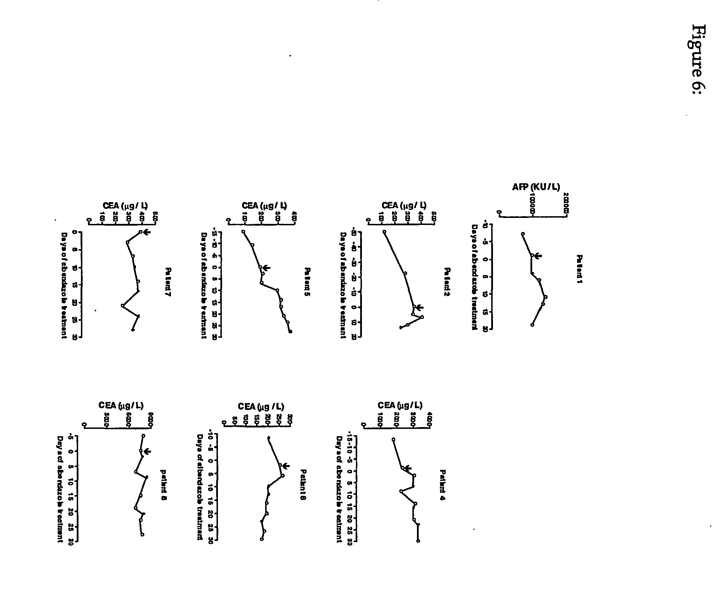

**Fig. 6****.** These figures are presented together with reference examples, for the useful understanding of the present invention. Serum tumor marker levels (AFP or CEA) in patients with liver tumors (CRC or HCC) under treatment with albendazole (10 mg / kg / day in two or three divided oral doses) for 28 days. Arrow indicates commencement of therapy.

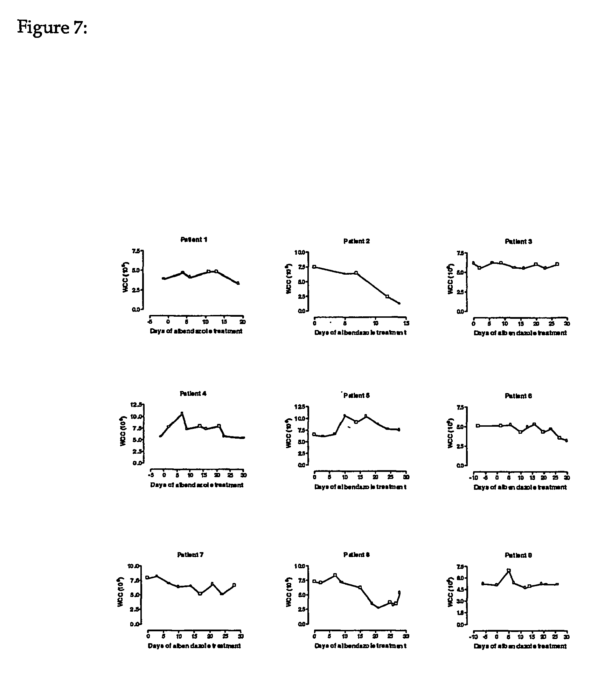

**Fig. 7****.** These figures are presented together with reference examples, for the useful understanding of the present invention. Serum white cell count (WCC) in patients with liver tumors (CRC or HCC) under treatment with albendazole (10 mg /kg / day in two or three divided oral doses) for 28 days.

The composition for use in the present invention comprises albendazole: 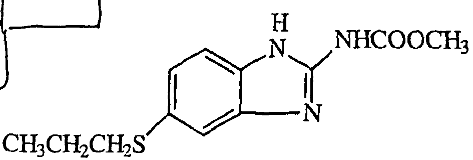 or a sulphoxide or sulphone thereof. The composition is for use in a method of treatment of an ovarian cancer in a subject.

The method may be used to treat primary or secondary cancers.

The method may include concomitant treatment with a potentiator of the benzimidazole compound effect on the cancer. The potentiator may be an isoquinoline compound (eg praziquantel) or any other compound which will increase or add to the effectiveness of the drug.

Albendazole is poorly absorbed from the gastrointestinal tract and also rapidly undergoes extensive first pass metabolism. At all times after the administration of a 400 mg oral dose, the concentration of the unchanged drug has been below the detection limits (18). This is mainly because of the rapid and extensive metabolism of the drug in the liver. Hydrolysis of the carbamate moiety and oxidation of the sulphur atom, the alkyl side chain and the aromatic ring have all been observed to occur in man. Five major metabolites have been identified in the human urine of which albendazole sulphoxide is the major one. The sulphoxide is biologically active and contributes to the activity of the drug. It attains peak plasma concentrations of about 200-300 ng/ml and has a plasma half life of about 8-9 hours. Together with other metabolites, it is mainly excreted in the urine with a small amount being excreted in the faeces (18,19).

As a result of this extensive metabolism, the parent drug is virtually undetectable in the body, and its anthelmintic effect seems to be partly exerted by the unabsorbed portion left in the intestine and partly by the active sulphoxide metabolite formed in the liver. However, to be effective in the treatment of HCC a concentration of greater than 100 nM must be available in the immediate vicinity of the tumor cells which means that, to attain effective and sustained antitumor concentrations of albendazole, large and frequent doses must be administered.

Secondly, the use of the drug as an anthelmintic has been associated with a number of side effects including mild and transient epigastric distress, diarrhoea, nausea, dizziness, lassitude and insomnia in short term treatments and reversible low grade transaminase elevation, jaundice, gastro intestinal symptoms, alopecia, rash or pruritus and leukopenia have been reported in patients under 3 month treatment courses for hydatid disease. Long term toxicity studies in animals showed diarrhoea, anaemia, hypotension, marrow depression, liver function test abnormalities, and fetal toxicity, varying by species (13).

The present inventors believe that regional administration of the benzimidazole compound to the liver may resolve the above mentioned limitations in the employment of the drug in method of treatment of the present invention. The present inventors also believe that this benefit may also be obtained through regional delivery of the benzimidazole compound to ovarian cancer

Regional delivery of the benzimidazole compound is achieved by administering the compound in a pharmaceutically acceptable formulation. The composition may be administered as continuous infusion of a solution via a pump through the major artery of the diseased organ

The formulation comprises a lipid. Particularly preferred are lipids for which the tumor is avid so that high concentrations of the drug may be delivered to the tumor.

Preferably the lipid is an oil. Preferably the formulation comprises an iodised oil. A particularly preferred iodised oil is lipiodol, an iodinated ethyl ester of the poppy seed oil.

Compared to systemic administration, regional delivery using a lipid such as lipiodol allows achievement of higher drug concentrations in the tumor site while reducing the degree of exposure of other body organs to the unwanted effects of the drug and consequently reducing the number and the severity of side effects. In HCC this can be made even more selective and effective by choosing lipiodol as the vehicle for the drug delivery.

HCC is described below for the useful understanding of the present invention. When injected into the hepatic artery, the oil is retained by HCCs for several weeks to over a year but is cleared from the normal liver parenchyma within 7 days. Without wishing to restrict the present invention in any way, one of the hypotheses in attempting to explain lipiodol retention in HCCs suggests that these cells are unable to clear lipiodol because they lack a reticuloendothelial kupffer cell component. We have previously shown that *in vitro*, vitamin D compounds such as 1, 25-dihydroxyvitamin D3 dissolved in lipiodol produce a profound and sustained inhibitory effect on HepG2 cells when injected through the hepatic artery of tumor bearing rats, the drug is retained within the tumor (See WO 98/56337 and WO 99156697

On the basis of the present inventors experience with albendazole, lipiodol, and hepatoma cell lines, they believe that administration of albendazole dissolved in an oil such as lipiodol and administered through the intrahepatic artery, will lead to the sustained release of the drug from the oil within the tumor cells leading to sustained inhibition of proliferation of the tumor cells.

These unique characteristics of lipiodol coupled with the potency and lipid solubility of albendazole, makes the combination an attractive formulation for intrahepatic arterial administration in patients with HCC.

Preferably the benzimidazole compound is present in the composition in a concentration of at least about 0.1M. The concentration of the benzimidazole compound is preferably in the range of about 0.1 to about 10 M.

The composition of the invention may include a potentiator of the effect of the benzimidazole compound on the cancer. The potentiator may be, for example, praziquantel or any other compound which would increase the effectiveness of the drug, have an additive effect with it, or reduce its side effects.

Throughout this specification the word "comprise", or variations such as "comprises" or "comprising", will be understood to imply the inclusion of a stated element, integer or step, or group of elements, integers or steps; but not the exclusion of any other element, integer or step, or group of elements, integers or steps.

In order that the nature of the present invention may be more fully understood the invention will now be described with reference to the following non-limiting embodiments.

### REFERENCE EXAMPLE 1

### IN VITRO AND IN VIVO SUPPRESSION OF GROWTH OF HEPATOCELLULAR CARCINOMA CELLS BY ALBENDAZOLE

### Materials and Methods

### Cell Culture

HepG2, Hep3-B, Hep1-6, SKHEP-1, PLC/PRF/5, and HTC cells were obtained from European Collection of Cell Cultures (ECACC; U.K), Novikoff was obtained from Cancer Research Centre (DKFZ) Heidelberg, Germany. Cells were cultured in MEM or DMEM supplemented with 10% FBS, 50 units / ml penicillin, 50 units/ml streptomycin, 25 µg/ml amphotericin B (Gibco, Grand Island, NY) and maintained subconfluent at 37° C in humidified incubators containing 5% CO₂. Albendazole (Sigma, Australian subsidiary) was dissolved in absolute ethanol at concentrations that were 1000-fold higher than the final medium concentration.

### [³H]Thymidine Incorporation Assay

For the study of [³H]thymidine incorporation, adherent cells (5-10 x 10⁴) were plated onto 24-well Corning tissue culture dishes and were exposed to culture medium (5% FBS) containing the vehicle (0.1% ethanol) or different concentrations of albendazole (10⁻⁸ to 10⁻⁶ M). For Novikoff, a detached rat cell line, 2500 cells were suspended in 2 ml of DMEM (5%FBS) and kept under the same condition as for attached cells. Media were replaced with fresh media on alternate days. At the end of the treatment period (5 days), cell cultures were assayed for thymidine incorporation by the addition of 0.5µCi of [³H]thymidine (60 Ci/mmol. ICN Biochem, Irvine, CA) to each well for the last 4 h of culture. The amount of radioactivity incorporated into cells was determined using a β-scintillation counter. Results are presented as percentage [³H]thymidine incorporation relative to control. For the recovery experiments, SKHEP-1 cells were treated for 1 day with different concentrations of albendazole and then either assayed for [³H]thymidine incorporation or the medium was replaced and the cells treated with fresh medium with out the drug for a further 4 days at the end of which, [³H] thymidine incorporation assay was performed.

### Cell Counts

SKEP-1 cells (2.5 x 10⁴) were plated in six well plates. The cell treatment procedure was as described for the thymidine assay. At the end of the treatment period (1, 3 or 5 days), cells were trypsinized and counted with a hemocytometer using the trypan blue exclusion method. In all experiments, cells treated with the medium containing 0.1% ethanol were taken as the control for albendazole treated cells. All counts were obtained in quadruplicate and each experiment was repeated at least twice.

### Cell Cycle Analysis

SKHEP-1 cells (5 x 10⁴) were plated onto six-well tissue culture plates. Triplicate samples were treated with the indicated concentrations of albendazole (100, 250 and 1000 nM). The medium was changed everyday. After 72 h the relevant group of cells were collected, washed twice with phosphate buffer and treated with ribonuclease, Triton X-100 and propidium iodide (Sigma) based on the method described by Taylor [12]. The percentage of cells within the G1, S, and G2-M phases of the cell cycle were determined using a FACScan flow cytometer (Becton Dickinson FACSort) and Multifit LT cell cycle analysis software (Verity Software INC.)

### Tumor formation in nude mice

6 to 10 weeks old male BALB/c Nu/Nu mice (Animal Resources Center, Perth, Australia) were inoculated subcutaneously with 10⁶ SKHEP-1 cells into the right flank. 24 hours after inoculation animals were randomly assigned to one of the treatment groups (n=10 per group), receiving 25, 50 or 150 mg/kg twice daily oral albendazole suspended in sesame oil for 20 days.

The control group was treated with the vehicle (sesame oil). Using vernier calipers, tumor diameter (mm) was measured on day eight and then every three days up to day 20-post tumor cell inoculation. Tumor volumes were calculated using the formula: ab²/ 2, where a and b are the smaller diameters in millimeters, respectively [13] and a piece of the tumor was preserved in paraffin for immunohistochemical determination of maximum proliferation index (MPI). Here, after fixation, the specimen was processed for the detection of Ki-67 antigen with the monoclonal antibody MIB1 according to the method described by McCormick [15].

The animal model was chosen on the basis of SKHEP-1 being the most tumorigenic human liver cancer cell line in nude mice [16] and previous experience with the model [17].

### Statistical Analysis

Differences between different treatment groups were analyzed using ANOVA followed by Tukey's test. P values of less than 0.05 were considered to represent a significant difference.

### Results

### Inhibition of [³H] Thymidine incorporation by albendazole

[³H] Thymidine incorporation assay was used to determine the effect of albendazole on cell proliferation in a number of human (HepG2, Hep3-B, PLC/PRF/5, SKHEP-1), rat (HTC and Novikoff) and mice (Hep1-6) HCC cell lines. Results obtained show that, in all cell lines examined, albendazole effectively reduces thymidine incorporation (Table 1). When treated with the 100 nM concentration of albendazole, compared to other cell lines, SKHEP-1 demonstrated the highest level of sensitivity to albendazole (p<0.01 compared to control), while the rat cell line HTC was the least responsive of all. Treatment with the 1000 nM concentration of albendazole reduced thymidine incorporation to less than 20% of control values (p<0.001) in all cell lines and to less than 5% in SKHEP-1 and HepG2. Here again SKHEP-1 cells displayed the highest level of sensitivity to albendazole. In these cells, thymidine incorporation was reduced to 0.6 ± 0.1% of the control values corresponding to 99.4% inhibition. For this reason, SKHEP-1 was employed for all further investigations. Exposure of SKHEP-1 cells to different concentrations of albendazole for 1 day, revealed that, concentrations of 250 nM and over of albendazole still produce profound inhibition of thymidine incorporation (Fig. 1a). Removal of the drug and treatment of cells with the normal medium for a further 4 days led to the recovery of thymidine incorporation by the cells (Fig. 1b). Except for the 500 and the 1000 nM concentrations, cells exposed to all other concentrations of albendazole were able to recover from the inhibitory effect of the drug.

**Table 1: Effect of albendazole on [³H] thymidine incorporation in HCC cell lines.**

| Cell line | [albendazole] nM | | | |
|---|---|---|---|---|
| | 10 | 100 | 500 | 1000 |
| HepG2 | 93.1 ± 5.6 | 72.9 ± 6.3 | 12.2 ± 2.9 | 3.5 ± 0.4 |
| Hep3-B | 105.7 ± 8.6 | 68.5 ± 5.7 | 22.6 ± 1.8 | 9.3 ± 0.7 |
| SKHEP-1 | 89.6 ± 6.1 | 63.7 ± 3.1 | 4.5 ± 1.1 | 0.6 ± 0.1 |
| PLC/PRF/5 | 92.7 ± 4.7 | 69.4 ± 5.2 | 26.9 ± 2.7 | 18.4 ± 2.1 |
| Novikoff | 96.5 ± 8.8 | 71.6 ± 5.9 | 29.2 ± 3.3 | 10.3 ± 1.8 |
| HTC | 98.4 ± 7.5 | 86.0 ± 6.9 | 28.5 ± 2.2 | 11.4 ± 1.3 |
| Hep1-6 | 97.7 ± 4.3 | 79.5 ± 3.2 | 28.1 ± 2.5 | 5.6 ± 0.6 |

Cells were treated with different concentrations of albendazole (10-1000 nM) for 5 days at the end of which [³H]thymidine incorporation was measured. Values (% control) represent mean ± s.e.m. of several determinations.

### Albendazole inhibits proliferation of cells leading to a decline in cell number

Counting of viable cells treated with different concentrations of albendazole for 1, 3 or 5 days produced a dose dependent decline in the number of cells, showing the profound inhibition of proliferation of SKHEP-1 cells by the drug (Fig. 2).

This was evident from day 3 at the 500 and the 1000 nM concentrations. Compared to control, cells exposed to the 1000 nM concentration of the drug, were significantly reduced in number (p<0.001).

### Dose-dependent effect of albendazole on the cell cycle kinetics

Flow cytometric analysis of albendazole-treated cells, revealed that, the drug induces a dose-dependant effect on the cell cycle kinetics of SKHEP-1 HCC cells. Fig. 3 demonstrates the changes induced on the distribution of cells in the different phases of the cell cycle following 3 days treatment with different concentrations of albendazole. From this, it is dearly evident that exposure of cells to the 250 nM concentration causes accumulation of cells in the G0-G1 phase and associated with this was a reduction in the percentage of cells in both S and G2-M phases of the cell cycle. Changes induced by the 500 nM concentration of the drug were identical to those of the 250 nM concentration (data not shown). However, as depicted in the same figure, treatment of the cells with the 1000 nM concentration of albendazole leads to a totally different pattern of changes. Here, arrest and accumulation of cells in the G2-M phase of the cell cycle was accompanied by a dramatic reduction in percentage of cells in the G0-G1 phase, while percentage of cells in the S phase remained unchanged.

### Effect of albendazole on tumor growth in vivo

In control animals, SKHEP-1 tumors grew to a mean volume of 87.9 ± 12.3 mm³ at 20 days post inoculation. In animals receiving 50 and 150 mg / kg per day, tumor growth was slightly but not significantly retarded. However, tumor growth was profoundly suppressed in animals receiving the 300 mg / kg dose of albendazole (Fig. 4) with a mean tumor volume of 12.0 ± 7.8 (p<0.001). Results from the immunohistochemical analysis of tumors revealed that, tumors from animals receiving the 50 and 150 mg / kg dose of albendazole had reduced MPIs of 22.54 ± 1.53 (mean ± s.e.m.) and 13.36 ± 3.04 respectively compared to 34.2 ± 3.13 for the control. There was not enough tissue for the analysis of MPI in tumors of mice receiving the 300 mg / kg / day dose.

### Dose-dependent effect of albendazole on the cell cycle kinetics

Albendazole was also shown to exhibit dose dependent inhibition of proliferation of the ovarian cancer cell line (OVCAR-3) *in vitro,* (see Figure 5).

### Discussion

Results from the cell proliferation studies clearly demonstrated that all human, rat and mice liver cell lines examined are profoundly inhibited by albendazole. This was manifested by the significant reduction of thymidine incorporation following treatment with albendazole doses of 100 nM and over. Similarly treatment of SKEP-1 cells with albendazole led to a dose and time dependent reduction of cell number. The reason behind the higher sensitivity of SKHEP-1 to albendazole is not clear at this stage. However, lacking the enzymes needed for the conversion of the drug to less active or inactive metabolites, may partly account for this observation [14]. Flow cytometric analysis of the cell cycle revealed that, albendazole causes differential dose-dependent effect on the cell cycle kinetics of SKHEP-1. Accumulation of cells in the G0-G1 phase following treatment with albendazole concentrations of up to 500 nM with an associated decline in percentage of cells in S and G2-M phases, indicates that progression out of the G1 phase was blocked. Many natural triggers for programmed cell death, including glucocorticoid hormones act at G1-G0 transition and the cells die in a process described as 'premature aging' [18]. However, following treatment with the 1000 nM concentration of albendazole, the pattern of cell distribution was reversed, leading to the accumulation of cells in the G2-M phase of the cycle. This indicates that the primary effect of albendazole at this concentration may be mediated by a transition delay through G2-M or mitosis.

The data from work in nude mice suggests that, at the higher dose of the 300 mg/ kg/day, albendazole presumably reaches the necessary concentrations required to suppress tumor formation. The very high rate of metabolism of albendazole in mice and the poor blood supply to the subcutaneous tumor, are amongst a number of factors that could account for the high dose of the drug required to suppress tumor growth in these animals.

The MPI data also confirm the ability of albendazole to reduce tumor proliferation rate. The Ki-67 antigen used in this assay is tightly linked to proliferation and has been used in a large number of studies to estimate the growth fraction of tumors [15].

### REFERENCE EXAMPLE 2

### ALBENDAZOLE IN PATIENTS WITH ADVANCED MALIGNANCY

### Patients and Methods

The study was single-centre, open and non-controlled. Nine patients (8 male and 1 female) with either advanced CRC and hepatic metastasis or HCC were included in this study. One patient with neuroendocrine cancer and mesothelioma was also treated on a compassionate basis. The patients aged between 38-79 years were inoperable and had failed existing chemotherapy and also, except for two, had measurable and increasing tumor markers. The majority had also already failed hepatic artery chemotherapy. The diagnosis of CRC or HCC was made by ultrasound, CT or MRI scan, confirmed by histology and by determination of CEA or AFP levels for CRC or HCC respectively. Only patients with expected survival of more than one month were enrolled into the study. Patient characteristics are presented in Table 2. The study was approved by the Human Ethics Committee for Research of SESAHS. The protocol and the aim of the study was dearly explained to each patient and informed consent obtained. The duration of this study was four weeks but was to be stopped if leukopenia (WBC < 2 x 10⁶) or severe hepatocellular injury (ALT or AST 2 x upper limit) developed. All patients received albendazole (400 mg scored tablets, Smith Kline Beecham, Australian subsidiary) 10 mg / kg orally in two or three divided doses for a planned duration of four weeks. This is the clinical dose of albendazole employed in the treatment of parasitic diseases. Patients were evaluated every three days by clinical examination together with full blood tests to monitor tumor markers, hematopoesis, liver and kidney function toxicity. A partial response (PR) was defined as a 50% or more decrease in the value of the markers. In addition there must be no new lesions or progression of any other lesions. Stable disease was defined as a decrease of less than 50%, or an increase of less than 25% in the value of tumor markers, while progressive disease (PD) was a 25% or more increase in the value of the tumor markers or the appearance of any new lesions.

**Table 2. Characteristics of the 9 patients with inoperable liver tumors, who had failed chemotherapy, participating in the phase 1 trial of albendazole.**

| Patient | Sex | Age | Type | Length of Treatment (days) | Comments | Site of metastatic disease |
|---|---|---|---|---|---|---|
| 1 | F | 38 | HCC | 19 | neutropenia; drug Withdrawn | MBL, lung & liver |
| 2 | M | 66 | CRC | 14 | neutropenia; drug Withdrawn | Liver & lung |
| 3 | F | 62 | * | 28 days | | Pleura & liver |
| 4 | M | 66 | CRC | 28 days | | MBL & bone |
| 5 | M | 56 | CRC | 28 days | | MBL & brain |
| 6 | M | 66 | CRC | 28 days | | Liver & lung |
| 7 | M | 74 | CRC | 28 days | | Liver & lung |
| 8 | M | 54 | CRC | 28 days | neutropenia Withdrawn | MBL |
| 9 | M | 79 | CRC | 28 days | | MBL& peritoneu m |

| | | | | | | |
|---|---|---|---|---|---|---|
| MBL = multiple bilobar liver * Mesothlioma and carcinoid tumor. | | | | | | |

### Results

Treatment with albendazole led to stabilization of the disease in three, and progression of the disease in the other two patients (Figure 6). In the remaining four, either the drug had to be withdrawn (2) or the tumor markers were not measurable (2). In patient number 1 suffering from HCC, albendazole treatment led to the stabilization of the disease, but because of the development of neutropenia, drug treatment was stopped in this patient on day 19. The patient visited another city after stopping albendazole and died of neutropaenic sepsis, which was almost certainly related to albendazole therapy. He had suffered neutropenia with previous chemotherapy. Patient number 2 (CRC), who was showing the greatest response to albendazole therapy, also developed neutropenia and so albendazole was also withdrawn in this patient. Patient number 3 (carcinoid tumor and mesothelioma) did not have evaluable serum tumor markers. However, the patient was monitored for adverse effects. In patients 4 and 5 there may have been a short term control of tumor markers but these began to rise again during treatment. In patients 6, 7 and 8, albendazole therapy was associated with stable CEA levels. In patient 9, the CEA levels were less than 10 µg / L and remained so for the duration of the treatment (4 weeks).

There were no significant changes in liver and kidney function tests during the course of the trial. However, in patients 1, 2 and 8 significant neutropenia developed as a result, of which the drug had to be withdrawn. WCC values for all nine patients are presented in Figure 7.

### Discussion

To the present inventors' knowledge, this is the first reported study of albendazole (or any other BZ) administered to human subjects for the therapy of cancer. Patients participating in this study had advanced malignancy, which had not responded to available therapy. Administration of albendazole to the only patient in the study with primary HCC led to the stabilisation of the AFP values. However, due to neutropenia, the drug had to be withdrawn. The patient had a recent history of low WCC. In the remaining patients (all with CRC) with measurable CEA levels, three had their tumor markers stabilised while on albendazole treatment. Compared to other patients, patient number two, who was withdrawn from the trial on day 19, had the steepest fall in CEA levels.

The present report demonstrates for the first time that, albendazole, a benzimidazole carbamate with extensive clinical use as a safe antiparasitic drug, can cause tumor marker stabilisation in patients with HCC or CRC with liver metastasis.

### EXAMPLE 3

To study the feasibility of using albendazole in the treatment of peritoneal disease, the human ovarian cancer cell line NIH:OVCAR-3 was selected. This is a cell line that grows quite slowly but is tumorogenic in nude mice therefore allowing for the drug to be studied under both *in-vitro* and *in-vivo* conditions.

### Thymidine assay

After finding the right conditions for the growth of cells in culture, cells were treated for 5 days with various concentrations of albendazole.

It was shown that concentrations as low as 0.001 micromoles/L of albendazole have an effect on the cell proliferation and that at a concentration of 0.25 micromoles/L, around 90% cell inhibition is achieved and at 0.5 micromoles/L inhibition of cell growth is 100%.

These results show that, albendazole is very effective against this human ovarian cancer cell. The degree of activity of albendazole is about X 10 more than that observed for liver or colorectal cancer cells where inhibitory activity starts at around 0.1 micromoles/L.

### Cell count

Cells were treated with either the medium alone (control) or 0.1 and 1 micromoles/L of albendazole for 0, 1, 3, 6 or 10 days and the number of viable cells counted. It was observed that at both doses employed albendazole treatment leads to profound reduction in cell number. This was evident from day 1 after the treatment.

### REFERENCE EXAMPLE 4

Albendazole/ albendazole sulfoxide against liver, colorectal and prostate cancer cell lines:

Animal and human studies have shown rapid conversion of albendazole to a sulphoxide (ABS) and then to a sulphone and a number of other metabolites. As an antiparasite, ABS is considered to be responsible for most or some of the systemic biological activity of albendazole, whereas, the sulphone metabolite is pharmacologically inactive. Several studies have shown that ABS is almost equally effective against parasites as the albendazole itself. Furthermore it is believed that the activity in the clinical setting is mainly due to the formation of ABS. So much so that ABS has now been considered as a new drug (ricobendazole) by some investigators. This knowledge together with some of our observations in the colorectal patients undergoing albendazole therapy, led us to test ABS against cancer cells. To date, there has been no literature on this issue.

In order to be able to test ABS, a proper solvent had to be found. This is because unlike albendazole ABS is not soluble in ethanol. After a number of tests, DMSO (0.5%) was found to be the most suitable solvent for this compound and for this type of investigation. Higher DMSO concentrations were found to be too toxic to the cells while lower concentrations led to incomplete solubility of ABS. Therefore to allow direct comparison between the 2 compounds, all subsequent experiments were run using 0.5% DMSO for both ABS and albendazole. In the cell lines examined, albendazole produced very similar results to what we had previously reported for the drug in liver cancer cells.

Both albendazole and ABS were tested side by side against various cell lines as follows:
Liver---HepG2, Hep3B, SKHEP-1
Colorectal---C-170, LOVO, HT-29
Prostate---PC3, LNCap, Du145
Leukeamic---HL60

The fist 9 cell lines were used to compare effectiveness and potency while, the last one was used to assess toxicity on bone marrow (as a rough guide).

Cells were treated with various concentrations of each compound for a period of 5 days.

Albendazole sulfoxide was shown to inhibit proliferation of human liver cancer cells HepG2, SKHEP-1 and Hep 3B.

ABS produced the same sort of activity against colorectal cell lines C-170, LOVO and HT-29. In general results obtained for ABS versus albendazole show a great degree of similarity between the 2 compounds with antiproliferative activity becoming evident at 0.01 micromoles/L concentration and dramatically increasing at 0.25 micromoles / L and nearing 100 inhibition at 1 micromoles / L.

Results obtained with prostate cell lines all demonstrate that, the drug and its major metabolite (ABS) are almost equipotent in inhibiting the proliferation of the human prostate cancer cells PC3, LNCap and Du 145.

### REFERENCE EXAMPLE 5

### Laddering:

To find out the way albendazole works as an anticancer drug, a large variety of tests can be performed. One of the first is to see if albendazole induces apoptosis. This again is tested in several ways, one of the most common is to check for DNA laddering. In our laboratories, after spending some time to find the right concentration and the right period of treatment, finally we could see dear evidence of induction of DNA laddering in these ovarian cancer cells by albendazole. This shows that, albendazole treatment leads to apoptosis. This is the first time that the induction of apoptosis by albendazole has been reported.

Similar results were also obtained with the human colorectal cell line LOVO. DNA obtained from LOVO cells treated with 1 µM albendazole for 16 hours showed dear laddering demonstrating that albendazole induces apoptosis in LOVO cells.

### Summary

Results obtained with the ovarian cell line (NIH:OVCAR-3) show for the first time that, albendazole is profoundly effective in inhibiting its proliferation in culture in a dose-dependent fashion. This was confirmed by both thymidine uptake and cell count. Further work clearly shows that these cells undergo apoptosis as a result of albendazole treatment.

Results obtained in liver and colorectal cell lines confirm the antiproliferative activity of albendazole against these cell lines. Results obtained with the 3 human prostate cell lines indicate that, these cells too, are quite sensitive to the effect of albendazole in culture. There is no previous report on the antiproliferative activity of albendazole in prostate cell lines making this the first to show such an activity.

It is well known for years that, conversion of albendazole in the body takes place quite rapidly in the liver and yields an active metabolite namely albendazole sulfoxide, which has been shown to be active against parasites. In this respect, our earlier results with the sulfoxide has shown it to be as effective as the parent drug (albendazole) in the 10 different cell lines examined. However, our latest results castes some doubt over this, and the compound is now under intensive investigation in our laboratories.

### REFERENCE EXAMPLE 6

### Combination therapy:

The effect of inhibition of metabolism of albendazole on its antiproliferative activity was investigated using human colorectal cell line LOVO and the methimazole as an inhibitor of albendazole

5000 cells each of HepG2 and SKHEP-1 were plated into 24 wells plates and treated for 5 days with various concentrations of albendazole alone or together with MT.

There was no growth inhibition of HepG2 cells when treated with albendazole for 5 days at the doses (0.25, 0.1 and 0.01uM) tested in this experiment. However, there was modest growth inhibition when albendazole and MT were used together. On the other hand, albendazole at 0.25 µM concentration profoundly inhibited the growth of SKHEP-1 cells. This response was further enhanced in the presence of MT. MT is an antithyroid agent which acts as a competitive inhibitor of microsomal Flavin Mono-oxygenase (FMO) dependent oxidation of several drugs. MT has been shown to inhibit the metabolism of drugs dependant on this pathway. MT mediated inhibition of liver sulfoxidation of albendazole to albendazole sulfoxide has been shown both *in-vitro* and *in-vivo.*

### References:

1. Akriviadis, E.A., Llovet, J.M., Efremidis, S.C. Hepatocellular carcinoma. Br. J. Surg. 85:1319-31,1998.
2. Mathurin, P., Rixe, D., Carbonell, N. et al. Review article: Overview of medical treatments in unresectable hepatocellular carcinoma - an impossible meta analysis? Aliment. Pharmacol. Ther. 12: 111-26, 1998.
3. Villa, E., Camollini, L., Dugani, A. et al. Variant estrogen receptor messenger RNA species detected in human primary hepatocellular carcinoma. Cancer Res. 55: 498-500, 1995.
4. Farmers, D. G., Rosove, M.H., Shaked, A. et al. Current treatment modalities for hepatocellular carcinoma. Ann. Surg. 219: 236-47, 1994.
5. Manesis, E. K., Giannoulis, G., Zoumboulis, P. et al. Treatment of hepatocellular carcinoma with combined suppression and inhibition of sex hormones: randomized controlled trial. Hepatology 21: 1535-42, 1995.
6. Okada, S. Chemotherapy in hepatocellular carcinoma. Hepatogastroenterology 45: 1259-63, 1998.
7. Kemeny, N. The systemic chemotherapy of hepatic metastases. Semin. Oncol. 10: 148-58, 1983.
8. Alvarez, L. I., Sanchez, S. F., Lanusse, C.E. In vivo and ex vivo uptake of albendazole and its sulfoxide metabolite by cestode parasites: relationship with their kinetic behaviour in sheep. J. Vet. Pharmacol. Therap. 22: 77-86, 1999.
9. Dollery, C (ed.) Therapeutic drugs, pp A31-A34; Churchil-Livingstone, 1996.
10. Lacey, E. The role of cytoskeletal protein, tubulin, in the mode of action and mechanism of drug resistance to benzimidazoles. Int. J. Parasitol. 18: 885-936,1988.
11. Lacey, E. The mode of action of benzimidazoles. Prasito. Today 6:112-115, 1990.
12. W. Taylor A rapid single step staining technique for DNA analysis by flow microfluorimetry. J. Histochem. Cytochem. 28 (1980) 1021-1024.
13. H. Yamamoto, J. Fujimoto, E. Okamoto, J. Furuyama, T. Tamaoki, T. Hashimoto-tamaoki, Suppression of growth of hepatocellular carcinoma by sodium butyrate in vitro and in vivo. Int. J. Cancer 76 (1998) 897-902.
14. S. Rolin, H. Souhaili-El Amri, A. M. Batt, M. Levy, D. Bagrel, G. Siest, Study of the in vitro bioactivation of albendazole in human liver microsomes and hepatoma cell lines. Cell Biol. Toxicol. 5 (1989) 1-14.
15. D. McCormic, H. Chong, C. Hobbs, C. Datta, P. A. Hall, Detection of the Ki-67 antigen in fixed and wax embedded sections with the monoclonal antibody MIB1. Histopathology 22 (1993) 355-360.
16. D. Shouval, L. Schuger, L.I. S. Levij, L.M. Reid, Z. Neeman, D. A. Shafritz, Comparative morphology and tumourigenicity of human hepatocellular carcinoma cell lines in athymic rats and mice. Virchows Arch. A Pathol. Anat. Histopathol. 412 (1988) 595-605.
17. M. H. Pourgholami, J. Akhter, Y. Lu, D. L. Morris, In vitro and in vivo inhibition of liver cancer cells by 1, 25-Dihydroxyvitamin D3. Cancer Lett. 151 (2000) 97-102.
18. L. A. Smets, Programmed cell death (apoptosis) and response to anti-cancer drugs. Anticancer Drugs 5 (1994) 3-9.

## Claims

1. A pharmaceutical composition for use in a method of treatment of an ovarian cancer in a subject by regional delivery,
wherein the composition comprises a lipid carrier and a benzimidazole carbamate which is albendazole or a sulfoxide or sulphone thereof.

2. A pharmaceutical composition for use according to claim 1 in which the carrier is an oil.

3. A pharmaceutical composition for use according to claim 2 in which the carrier is an iodised oil.

4. A pharmaceutical composition for use according to claim 3 in which the iodised oil is an iodinated ethyl ester of the poppy seed oil.

5. A pharmaceutical composition for use according to anyone of claims 1 to 4 in which the composition further comprises methimazole.

## Patentansprüche

1. Pharmazeutische Zusammensetzung zur Verwendung in einem Verfahren zur Behandlung von Ovarialkrebs in einem Individuum durch regionale Verabreichung,
wobei die Zusammensetzung einen Lipidträger und ein Benzimidazolcarbamat umfasst, das Albendazol oder ein Sulfoxid oder Sulfon davon ist.

2. Pharmazeutische Zusammensetzung zur Verwendung nach Anspruch 1, wobei der Träger ein Öl ist.

3. Pharmazeutische Zusammensetzung zur Verwendung nach Anspruch 2, wobei der Träger ein iodiertes Öl ist.

4. Pharmazeutische Zusammensetzung zur Verwendung nach Anspruch 3, wobei das iodierte Öl ein iodierter Ethylester von Mohnöl ist.

5. Pharmazeutische Zusammensetzung zur Verwendung nach einem der Ansprüche 1 bis 4, wobei die Zusammensetzung weiters Methimazol umfasst.

## Revendications

1. Composition pharmaceutique pour utilisation dans une méthode de traitement d'un cancer des ovaires chez un sujet par délivrance régionale,
où la composition comprend un support de lipides et un benzimidazole carbamate qui est l'albendazole ou un sulfoxyde ou sulfone de celui-ci.

2. Composition pharmaceutique pour utilisation selon la revendication 1, où le support est une huile.

3. Composition pharmaceutique pour utilisation selon la revendication 2, où le support est une huile iodée.

4. Composition pharmaceutique pour utilisation selon la revendication 3, dans laquelle l'huile iodée est un éthyl ester iodé de l'huile d'oeillette.

5. Composition pharmaceutique pour utilisation selon l'une quelconque des revendications 1 à 4, où la composition comprend en outre du méthimazole.
